# EUROPÄISCHE PATENTANMELDUNG

(11) **EP 2 846 138 A2**
(43) Veröffentlichungstag der Anmeldung: **11.03.2015**
(21) Anmeldenummer: 14183217.0
(22) Anmeldetag: 02.09.2014
(51) Int. Cl.: G01D 11/24

(54) **Sondenhalter für Fließgewässer**

(30) Priorität: 05.09.2013 DE 102013217797
(71) Anmelder: Helmholtz-Zentrum für Umweltforschung GmbH-UFZ, 04318 Leipzig (DE)
(72) Erfinder: Keller, Toralf, 04249 Leipzig (DE); Oosterwoud, Marieke, 04317 Leipzig (DE)
(74) Vertreter: Gulde & Partner

(57) **Zusammenfassung**

Die Erfindung betrifft einen Sondenhalter (1) zur Fixierung einer zylindrischen Sonde (5) in einem Fließgewässer. Um die Sonde (5) sicher im Fließgewässer halten zu können und dabei ein störungsfreies Anströmen der Sonde (5) zu ermöglichen, weist der Sondenhalter (1) ein offenes Strömungsfenster (7) auf.

## Beschreibung

Die Erfindung betrifft einen Sondenhalter zur Fixierung einer zylindrischen Sonde in einem Fließgewässer.

Sondenhalter der oben genannten Art sind allgemein bekannt. Sie werden dazu verwendet, um eine Sonde in einem Fließgewässer stationär zu halten, wobei die Sonde beispielsweise zur Überwachung der Wasserqualität des Fließgewässers verwendet wird. Insbesondere finden optische Spektrometersonden, die durch das Fließgewässer geleitetes Licht im visuellen oder im ultravioletten Spektrum zur Bestimmung der Wasserqualität verwenden, Anwendung. Die Anbringung der Sonde in bekannten Sondenhaltern ist jedoch aufwendig, da die Sonde sowohl stationär gehalten, einfach im Fließgewässer installiert, als auch gut vom Wasser des Fließgewässers umströmt werden soll.

Es ist daher die Aufgabe der Erfindung, einen Sondenhalter bereitzustellen, der einfach anwendbar ist und zylindrische Sonden sicher stationär hält, ohne die Bestimmung der Wasserqualität zu beeinträchtigen.

Diese Aufgabe wird für den eingangs genannten Sondenhalter erfindungsgemäß durch einen Aufnahmekanal für die Sonde gelöst, wobei sich der Aufnahmekanal durch ein in und entgegen einer Längsrichtung des Aufnahmekanal begrenztes Strömungsfenster des Sondenhalters erstreckt, das quer zur Längsrichtung offen ist.

Das offene oder durchgängige Strömungsfenster ermöglicht, dass das Wasser des Fließgewässers die Sonde direkt umströmen kann, ohne dass der Sondenhalter im Bereich eines Sensorelements der Sonde eine Barriere für das fließende Wasser bildet. Folglich kann die Wasserqualität des Fließgewässers jederzeit aktuell ermittelt werden, da immer frisches Wasser des Fließgewässers an der Sonde vorbeiströmt, wenn die Sonde mit dem Sondenhalter im Fließgewässer angeordnet ist

Die erfindungsgemäße Lösung kann durch verschiedene, jeweils für sich vorteilhafte, beliebig miteinander kombinierbare Ausgestaltungen weiter verbessert werden. Auf diese Ausgestaltungsformen und die mit ihnen verbundenen Vorteile ist im Folgenden eingegangen.

So kann sich der Aufnahmekanal durch zwei voneinander beabstandet angeordnete hohlzylindrische Sondenaufnahmen erstrecken. Die voneinander beabstandet angeordneten Sondenaufnahmen halten die Sonde sicher und gewährleisten, dass das im Fließgewässer strömende Wasser die Sonde nicht bewegt oder mitreißt. Aufgrund des Abstandes der Sondenaufnahmen zueinander blockiert der Sondenhalter das herumströmende Wasser im Bereich des Strömungsfensters nicht.

Die Sondenaufnahmen können entlang der Längsrichtung des Aufnahmekanals hintereinander angeordnet sein. Die Sonde kann also einfach entlang der Längsrichtung in den Aufnahmekanal und in die beiden Sondenaufnahmen eingeschoben werden. Insbesondere wenn die Sondenaufnahmen entlang der Längsrichtung miteinander fluchten und den Aufnahmekanal vorzugsweise quer zu seiner Längsrichtung begrenzen, ist die Sonde besonders einfach in den Aufnahmekanal einsetzbar.

Die hohlzylindrischen Sondenaufnahmen weisen jeweils einander gegenüberliegende Enden auf, wobei durch ein Ende einer der Sondenaufnahmen die zylindrische Sonde in den Aufnahmekanal einschiebbar ist. Ein von der anderen Sondenaufnahme weg weisendes Ende einer der Sondenaufnahmen kann geschlossen sein. Das geschlossene Ende der einen Sondenaufnahme kann einen Anschlag für die Sonde ausbilden, sodass die Sonde parallel zur Längsrichtung optimal positioniert werden kann.

Die von der jeweils anderen Sondenaufnahme weg weisenden Enden der Sondenaufnahmen können gegen ein ungewolltes Öffnen gesichert verschlossen sein. Insbesondere kann eines der Enden dauerhaft verschlossen und ein anderes der Enden temporär geschlossen oder schließbar sein. Das dauerhaft geschlossene Ende kann den Anschlag für die Sonde ausbilden. Durch das auch wiederholt zu öffnende- und zu verschließende Ende kann die Sonde in den Aufnahmekanal eingesetzt werden. Ist das schließbare Ende gegen ungewolltes Öffnen gesichert und beispielsweise verschlossen, kann nicht nur die Sonde unverlierbar im Aufnahmekanal aufgenommen und/oder gehalten sein. Vielmehr kann die Sonde auch gegen Diebstahl (z.B. durch Sicherungsschrauben oder ein wasserdichtes Vorhängeschloss) geschützt im Aufnahmekanal angeordnet sein.

Zumindest eines der verschlossenen Enden und insbesondere das verschließbare Ende kann wenigstens eine Durchführung für eine Anschlussleitung der Sonde aufweisen.

Zur ortsfesten Positionierung der Sonde kann der Sondenhalter ein Abstellelement aufweisen, das mit den Sondenaufnahmen starr verbunden ist und auf dem der Sondenhalter verkippsicher abstellbar ist. Beispielsweise kann das Abstellelement ausgebildet sein, in einen Grund des Fließgewässers hineingerammt zu werden. Um den Grund des Fließgewässers nicht unnötig zu beschädigen oder wenn der Grund beispielsweise bei einem künstlich angelegten Kanal zu fest ist, um das Abstellelement dort hinein zu rammen, kann das Abstellelement auch als ein stabil auf eine Fläche stellbares Abstellelement ausgebildet sein. In einer besonders einfachen Ausgestaltungsform kann das Abstellelement als eine Abstellplatte ausgebildet sein.

Um zu verhindern, dass das Fließgewässers den Sondenhalter bewegt oder gar wegspült, kann das Abstellelement eine Ablagefläche für Schüttgut aufweisen, die den Sondenaufnahmen zugewandt ist. Beispielsweise kann Sediment vom Grund des Fließgewässers auf die Ablagefläche aufgebracht werden oder das Abstellelement in den Grund des Fließgewässers eingegraben werden, um den Sondenhalter im Fließgewässer zu fixieren.

Zur Sicherung des Sondenhalters im Fließgewässer kann das Abstellelement wenigstens eine durchgängige Öffnung aufweisen, die sich vorzugsweise beabstandet von Rändern des Abstellelements vollständig durch das Abstellelement erstreckt. An der Öffnung kann ein Ankerelement befestigbar sein. Alternativ kann der Sondenhalter mit einem Haltestift bereitgestellt werden, der durch die durchgängige Öffnung hindurch in den Grund des Fließgewässers einsteckbar ist, um des Sondenhalter zu fixieren. Der Haltestift kann beispielsweise ein sogenannter Zeltnagel sein. Insbesondere kann das Abstellelement mehrere voneinander beabstandete Öffnungen und beispielsweise zwei, drei, vier oder mehr Öffnungen aufweisen, um den Sondenhalter auch bei stark strömenden Fließgewässern sicher fixieren zu können. Auch ein Seil und insbesondere ein Edelstahlseil kann an der Öffnung befestigt sein, um den Sondenhalter zu sichern.

Zur Verbindung zumindest einer der Sondenaufnahmen mit dem Abstellelement kann der Sondenhalter ein Verbindungselement aufweisen, an dem zumindest eine der Sondenaufnahmen und das Abstellelement befestigt sind. Um zu vermeiden, dass vom Grund des Fließgewässers aufgewirbelte Partikel die Bestimmung der Wasserqualität beeinträchtigen, ist die zumindest eine Sondenaufnahme vorzugsweise beabstandet vom Abstellelement am Verbindungselement befestigt. Das Verbindungselement ist beispielsweise als eine Verbindungsrippe oder als eine Verbindungsplatte ausgebildet, wobei die zumindest eine Sondenaufnahme an einen Rand des Verbindungselementes und das Abstellelement an einem der Sondenaufnahme gegenüberliegenden Rand des Verbindungselementes befestigt sind. Insbesondere können beide Sondenaufnahmen am Verbindungselement befestigt sein.

Das Verbindungselement kann sich entlang der Längsrichtung des Aufnahmekanals erstrecken, um dem strömenden Wasser des Fließgewässers möglichst wenig Widerstand zu bieten. Parallel zur Längsrichtung kann das Verbindungselement eine Länge aufweisen, die einer Länge des Aufnahmekanals im Wesentlichen entsprechen kann. Ist jedoch beabsichtigt, dass das Fließgewässer bei der Bestimmung der Wasserqualiltät quer zum Aufnahmekanal strömen soll, so kann sich das Verbindungselement auch quer zur Längsrichtung erstrecken. Ist das Verbindungselement als eine Verbindungsplatte ausgebildet, so kann diese als ein Strömungselement die Ausrichtung des Sondenhalters im Fließgewässer vorgeben.

Beispielsweise weist das Verbindungselement einen an einer der Sondenaufnahmen anliegenden Halteabschnitt auf, an dem die Sondenaufnahme fixiert und mit dem die Sondenaufnahme beispielsweise verschweißt ist. Ferner weist das Verbindungselement vorzugsweise einen zwischen den Sondenaufnahmen angeordneten Strömungsabschnitt auf, wobei der Strömungsabschnitt einen größeren Abstand zum Aufnahmekanal aufweist, als der Halteabschnitt. Der Strömungsabschnitt grenzt an das sich bis zum Aufnahmekanal und zwischen den Sondenaufnahmen hindurch erstreckende Strömungsfenster an, wobei durch das Strömungsfenster Wasser des Fließgewässers ungehindert zur Sonde fließen kann. Vorzugsweise ist das Verbindungselement mit zwei Halteabschnitten ausgebildet, wobei sich das Strömungsfenster und womöglich auch der Strömungsabschnitt zwischen den beiden Halteabschnitten erstrecken.

Das Verbindungselement kann eine Öffnung zur Verbindung des Sondenhalters mit einer Verankerung aufweisen, um den Sondenhalter sicher positionieren zu können. Alternativ ist an der Öffnung auch ein Seil befestigbar, um den Sondenhalter aus dem Fließgewässer herausziehen und/oder gegen Diebstahl schützen zu können. Die Öffnung ist beispielsweise von Rändern des Verbindungselementes beabstandet und vorzugsweise näher zu einem im Betrieb angeströmten Rand als einem diesem gegenüberliegenden Rand angeordnet. Die Öffnung kann sich quer zu einer Längsrichtung des Verbindungselements, die der Längsrichtung des Aufnahmekanals entsprechen kann, vollständig durch das Verbindungselement erstrecken.

Um die mechanische Stabilität des Sondenhalters zu verbessern, kann dieser mit wenigstens einem Stützelement ausgebildet sein. Das Stützelement sichert mindestens eine der Sondenaufnahmen quer zum Aufnahmekanal gegen Bewegungen, insbesondere relativ zum Abstellelement Zum Beispiel durch Verwirbelungen im Fließgewässer hervorgerufene Kräfte, die auch quer zu einer Fließrichtung des Fließgewässers wirken können, werden durch das Stützelement aufgenommen, sodass die wenigstens eine Sondenaufnahme durch das Fließgewässer möglichst wenig bewegt wird.

Das Stützelement ist beispielsweise beabstandet vom Verbindungselement am Abstellelement und beabstandet vom Abstellelement am Verbindungselement befestigt. Das Stützelement, das Verbindungselement und das Abstellelement können somit quer zur Längsrichtung des Aufnahmekanals einen fachwerkartigen Aufbau aufweisen, durch den auf den Sondenhalter einwirkende Kräfte effizient aufgenommen und abgeleitet werden können. Insbesondere kann der Sondenhalter zwei Stützelemente aufweisen, die an sich gegenüberliegenden Seiten des Verbindungselementes angeordnet sind.

Auch die Stützelemente können plattenförmig und sich entlang der Längsrichtung des Aufnahmekanals oder entlang des Verbindungselements erstreckend ausgebildet sein, um dem Wasser des Fließgewässers einen geringen Strömungswiderstand zu bieten.

Ein Gehäuse der Sonde ist oftmals aus Metall gefertigt, um einen dauerhaften oder häufigen Einsatz der Sonde in Fließgewässern zu ermöglichen. Damit der Sondenhalter die Sonde stabil hält und ebenfalls eine lange Lebensdauer besitzt, können auch die Sondenaufnahmen aus einem Metall gefertigt sein. Um eine elektrochemische Korrosion der Sonde und/oder der Sondenaufnahmen im Fließgewässer zu vermeiden, kann der Sondenhalter einen Abstandshalter zur Positionierung der Sonde beabstandet zu einer Innenseite einer der Sondenaufnahmen aufweisen. Insbesondere können beide Sondenaufnahmen jeweils mit zumindest einem Abstandshalter versehen sein, sodass die Sonde metallische Bestandteile der Sondenaufnahmen nicht mechanisch kontaktiert. Der Abstandshalter ist vorzugsweise in wenigstens einer der Sondenaufnahmen angeordnet oder ragt in diese hinein. Der Aufnahmekanal kann quer zu seiner Längsrichtung an den Abstandshalter angrenzen und durch diesen beabstandet zur Innenseite der Sondenaufnahme verlaufen.

Der Abstandshalter kann als ein Formkörper mit einer Durchgangsöffnung ausgebildet sein. Insbesondere kann eine Außenseite des Abstandshalters zumindest abschnittsweise komplementär zu einer Innenseite einer der Sondenaufnahmen ausgebildet sein. Der Abstandshalter ist insbesondere in die Sondenaufnahme einsetzbar. Weist die Sondenaufnahme beispielsweise einen kreisförmigen inneren Querschnitt auf, so folgt die Außenseite des Abstandshalters zumindest teilweise einen Kreisbogen. Der Abstandshalter kann also zumindest abschnittsweise einen ringförmigen Querschnitt aufweisen oder als ein Ring ausgebildet sein.

Der Abstandshalter kann ferner ausgebildet sein, die Sonde verdrehsicher zu halten.

Im Folgenden ist die Erfindung beispielhaft anhand von Ausführungsbeispielen mit Bezug auf die Zeichnungen erläutert. Die unterschiedlichen Merkmale der Ausführungsformen können dabei unabhängig voneinander kombiniert werden, wie es bei den einzelnen vorteilhaften Ausgestaltungen bereits dargelegt wurde. Es zeigen:
- Figur 1: eine schematische Aufsicht eines ersten Ausführungsbeispiels des erfindungsgemäßen Sondenhalters;
- Figur 2: eine schematische Seitenansicht eines weiteren Ausführungsbeispiels des erfindungsgemäßen Sondenhalters; und

- Figur 3: eine schematische Vorderansicht eines weiteren Ausführungsbeispiels des erfindungsgemäßen Sondenhalters.

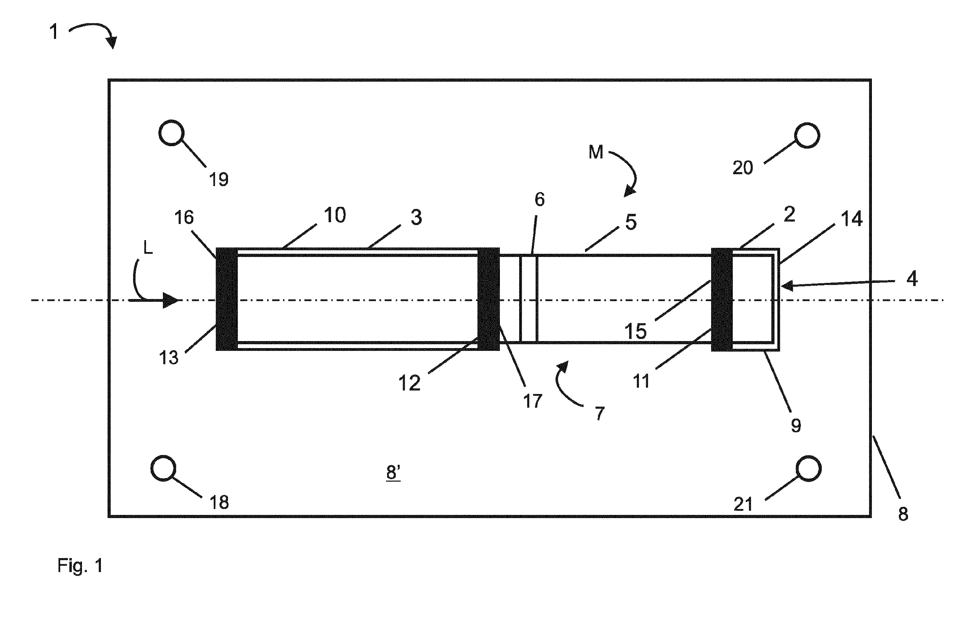

Figur 1 zeigt den Sondenhalter 1 schematisch in einer Aufsicht. Der Sondenhalter 1 des Ausführungsbeispiels der Figur 1 ist mit zwei Sondenaufnahmen 2, 3 versehen dargestellt, durch die sich entlang einer Längsrichtung L ein Aufnahmekanal 4 für eine zylindrische Sonde 5 erstreckt. Die Sondenaufnahmen 2, 3 sind in der Längsrichtung L hintereinander angeordnet. Die Sonde 5 ist in der Längsrichtung L in den Aufnahmekanal 4, also in die Sondenaufnahmen 2, 3, eingeschoben gezeigt. Durch die hohlzylindrische Ausbildung der Sondenaufnahmen 2, 3 ist die Sonde 5 gegen ungewollte Verschiebungen quer zur Längsrichtung L und gegen Verdrehen z. B. durch formschlüssige Abstandsthalter im Aufnahmekanal 4 gesichert.

Die Sonde 5 weist zumindest ein Sensorelement/Messfenster 6 auf, das zum Bestimmen der Wasserqualität des Fließgewässers mit dessen Wasser in Kontakt zu bringen ist. Dadurch, dass die Sondenaufnahmen 2, 3 entlang der Längsrichtung L voneinander beabstandet vorgesehen sind, erstreckt sich zwischen den Sondenaufnahmen 2, 3 ein Strömungsfenster 7, in dem zumindest das Sensorelement 6 der Sonde 5 anordenbar ist. Die Sondenaufnahmen 2, 3 hindern das Fließgewässer also nicht zum Sensorelement 6 der Sonde 5 zu strömen.

Damit der Sondenhalter 1 stabil ins Fließgewässer gestellt werden kann, weist der Sondenhalter 1 ein Abstellelement 8 auf. Das Abstellelement 8 ist beispielhaft als eine Abstellplatte gezeigt, die sich parallel und quer zur Längsrichtung L erstreckt. Eine auf den Aufnahmekanal 4 zu weisende Seite des Abstellelements 8 ist als eine Ablagefläche 8' für Schüttgut ausgebildet. Auf die Ablagefläche 8' aufgebrachtes Schüttgut kann zur Positionssicherung des Sondenhalters 1 im Fließgewässer dienen.

Um zu verhindern, dass die Sonde 5 beispielsweise metallische Hülsenabschnitte 9, 10 der Sondenaufnahmen 2, 3 direkt mechanisch kontaktiert, ist der Sondenhalter 1 mit drei Abstandshaltern 11, 12, 13 ausgestattet. Die Abstandshalter 11, 12, 13 sind vorzugsweise elektrisch isolierend und beispielsweise aus Kunststoff ausgebildet, um bei direktem Kontakt mit der Sonde 5 eine galvanische Korrosion eines metallischen Gehäuses der Sonde 5 zu vermeiden.

Zumindest eine der Sondenaufnahmen 2, 3 und beispielsweise die Sondenaufnahme 2 kann ein verschlossenes Ende 14 aufweisen, das eine Einstecktiefe der Sonde 5 in der Längsrichtung L in den Aufnahmekanal 4 begrenzt. Das verschlossene Ende 14 formt dabei einen Anschlag für die Sonde 5 aus, sodass diese in der Längsrichtung L nicht über ihre dargestellte Messposition M hinaus verschiebbar ist. In der Messposition M ist das Sensorelement 6 im Strömungsfenster 7 angeordnet. Das verschlossene Ende 14 weist vorzugsweise von der anderen Sondenaufnahme 3 weg.

Ein dem verschlossenen Ende 14 gegenüberliegendes Ende 15 ist vorzugsweise offen oder geöffnet, sodass die Sonde 5 einfach entlang der Längsrichtung der Sondenaufnahme 2 zunächst durch die Sondenaufnahme 3 und danach durch das offene Ende 15 der Sondenaufnahme 2 in den Aufnahmekanal 4 eingesetzt werden kann. Die Sondenaufnahme 2 weist zum Beispiel die Form eines Bechers auf, der sich entgegen der Längsrichtung L öffnet.

Die Sondenaufnahme 3 ist mit in und entgegen der Längsrichtung L weisenden Enden 16, 17 ausgebildet, die zumindest bei einem Einsetzvorgang der Sonde 5 in den Aufnahmekanal 4 offen sind, um ein einfaches Einsetzen der Sonde 5 zu gewährleisten.

Um zu verhindern, dass der Sondenhalter 1 durch das Fließgewässer weggespült wird, kann dessen Abstellelement 8 wenigstens eine Öffnung 18 aufweisen, durch die das Abstellelement 8 und somit der Sondenhalter 1 relativ zu einem Grund des Fließgewässers fixierbar ist. Die Öffnung 18 kann beispielsweise mit einem Ankerelement, das Teil eines den Sondenhalter umfassenden Bausatzes sein kann, verbindbar sein. Alternativ oder zusätzlich kann ein Haltestift, der ebenfalls Teil des Bausatzes sein kann, durch die Öffnung 18 in den Grund des Fließgewässers gesteckt werden, um ein ungewolltes Verschieben des Sondenhalters 1 relativ zum Grund zumindest zu erschweren oder sogar zu verhindern. Das Abstellelement 8 kann mit mehreren Öffnungen und beispielsweise mit vier Öffnungen 18 bis 21 ausgebildet sein, wobei die Öffnungen 18 bis 21 vorzugsweise voneinander beabstandet und insbesondere in Eckbereichen des zum Beispiel als eine rechteckig ausgebildete Abstellplatte ausgeformten Abstellelementes 8 angeordnet sein können.

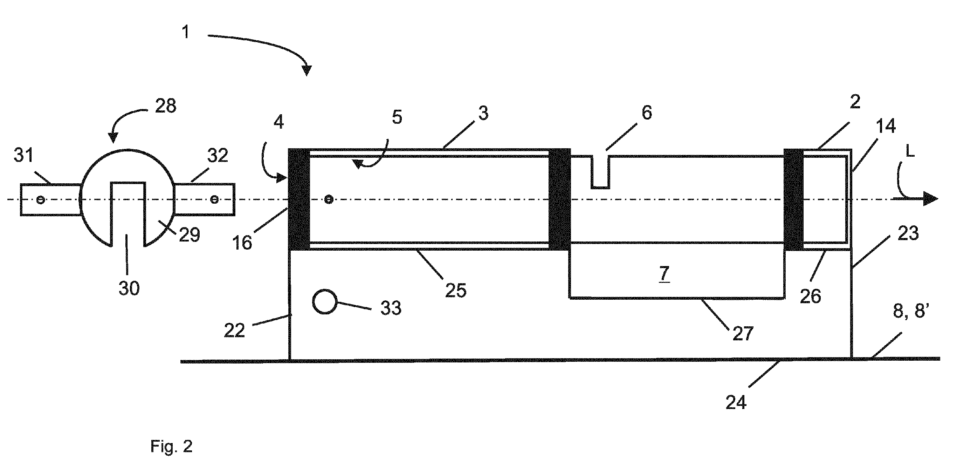

Figur 2 zeigt ein weiteres Ausführungsbeispiel des erfindungsgemäßen Sondenhalters 1 schematisch in einer Seitenansicht. Für Elemente, die in Funktion und/oder Aufbau den Elementen des Ausführungsbeispiels der Figur 1 entsprechen, sind dieselben Bezugszeichen verwendet.

Der Sondenhalter 1 des Ausführungsbeispiels der Figur 2 ist ebenfalls mit der im Aufnahmekanal 4 angeordneten Sonde 5 dargestellt. Zumindest eine der Sondenaufnahmen 2, 3 und insbesondere beide der Sondenaufnahmen 2, 3 sind mit Hilfe eines Verbindungselementes 22 starr mit den Abstellelement 8 verbunden. Beispielsweise sind die Sondenaufnahmen 2, 3 an einer quer zur Längsrichtung L weisenden Seite 23 des Verbindungselements 22 befestigt, insbesondere durch verschweißen. Eine der Seite 23 gegenüberliegende Seite 24 des Verbindungselementes 22 kontaktiert das Abstellelement 8 und ist beispielsweise durch verschweißen an diesem befestigt.

Das Verbindungselement 22 sorgt nicht nur für eine stabile Verbindung zwischen den Sondenaufnahmen 2, 3 und dem Abstellelement 8. Vielmehr weisen die Sondenaufnahmen 2, 3 durch das Verbindungselement 22 quer zur Längsrichtung L einen Abstand zu Abstellelement 8 auf. Steht der Sondenhalter 1 mit seinem Abstellelement 8 auf einem Grund des Fließgewässers, so sind die Sondenaufnahmen 2, 3 und hierdurch auch die Sonde 5 mit einem Abstand zum Grund im Fließgewässer angeordnet. Partikel des Grundes, die aufgrund von Strömungen des Fließgewässers womöglich aufgewirbelt werden, behindern aufgrund des Abstandes die Bestimmung der Wasserqualität weniger oder sogar gar nicht.

Das Verbindungselement 22 und insbesondere dessen Seite 23 weist wenigstens einen Halteabschnitt 25 und vorzugsweise zwei Halteabschnitte 25, 26 auf. Die Halteabschnitte 25, 26 kontaktieren jeweils eine der Sondenaufnahmen 2, 3 direkt. Zwischen den Sondenaufnahmen 2, 3 ist das Verbindungselement 22 mit einem Rücksprung 27 ausgebildet, der zum Aufnahmekanal 4 einen größeren Abstand aufweist, als die Halteabschnitte 25, 26. Da der Rücksprung 27 an das Strömungsfenster 7 angrenzt und ein Umströmen der Sonde 5 an allen Seiten quer zur Längsrichtung L ermöglicht, kann der Rücksprung 27 auch als ein rückspringender Strömungsabschnitt 27 des Verbindungselementes 22 bezeichnet sein.

Das Verbindungselement 22 erstreckt sich beispielsweise parallel zur Längsrichtung L und kann als ein einstückig handhabbares Teil und beispielsweise als eine Stanzteil gefertigt sein. Alternativ kann das Verbindungselement 22 aus mehreren und beispielsweise aus drei Teilen bestehen, wobei der Rücksprung 27 zwei weitere Teile des Verbindungselementes 22 miteinander verbindet.

Das Verbindungselement 22 ist vorzugsweise als eine einteilige oder mehrteilige Platte ausgebildet, die sich entlang der Längsrichtung L und quer zum Abstellelement 8 erstreckt. In einem strömenden Fließgewässer sorgt das plattenförmige Verbindungselement 22 für eine stabile Ausrichtung des Sondenhalters 1.

Soll die Sonde 5 nicht parallel zur Längsrichtung L vom Wasser des Fließgewässers angeströmt werden, kann das plattenförmige Verbindungselement 22 auch unter einem anderen Winkel zur Längsrichtung L und beispielsweise senkrecht dazu ausgerichtet sein. Um insbesondere bei einer senkrecht zur Längsrichtung L verlaufenden Ausrichtung des Verbindungselementes 22 beide Sondenaufnahmen 2, 3 mit dem Abstellelement 8 starr verbinden zu können, kann der Sondenhalter 1 zumindest zwei Verbindungselemente 22 aufweisen, welche jeweils eine der Sondenaufnahme 2, 3 starr mit dem Abstellelement 8 verbinden. Verlaufen die Verbindungselemente 22 unter einem Winkel größer null zur Längsrichtung L und insbesondere senkrecht zur Längsrichtung L, braucht das Verbindungselement 22 nicht mit dem Rücksprung 27 ausgebildet sein. Das Strömungsfenster 7 erstreckt sich dann zwischen den Sondenaufnahmen 2, 3 bis zum Abstellelement 8.

Um zu verhindern, dass die Sonde 5 entgegen der Längsrichtung L ungewollt aus dem Aufnahmekanal 4 herausgezogen werden kann, kann das von der Sondenaufnahme 2 weg weisende Ende 16 der Sondenaufnahme 3 verschließbar sein. Hierzu kann der Sondenhalter 1 einen Deckel 28 aufweisen, mit dem die Öffnung 16 zumindest abschnittsweise blockierbar ist.

Der Deckel 28 weist beispielsweise eine Verschlussplatte 29 auf, die im verschlossenen Zustand der Öffnung 16 diese in der Längsrichtung L wenigstens teilweise und vorzugsweise vollflächig abdeckt. Eine optionale Durführöffnung 30 der Verschlussplatte 29 ermöglicht ein Herausführen von Anschlusskabeln der Sonde 5 aus der Sondenaufnahme 3, sodass die Kabel nicht durch das Strömungsfenster 7 hindurchgeführt werden müssen. Die Durchführöffnung 30 ist beispielsweise als ein Schlitz, eine Nut oder als eine Kerbe ausgebildet und öffnet sich quer zur Längsrichtung L, um Anschlusskabel in sie einlegen zu können.

Zur Fixierung des Deckels 28 am Sondenhalter 1 kann dieser Fixierlaschen 31, 32 aufweisen. Die Fixierlaschen 31, 32 sind beispielsweise an der Verschlussplatte 29 schwenkbar angebracht, sodass die Fixierlaschen 31, 32 an einer quer zur Längsrichtung L weisenden Außenseite der Sondenaufnahme 3 anlegbar sind. Die Fixierlaschen 31, 32 weisen beispielsweise jeweils eine Öse auf, um den Deckel 28 an der Sondenaufnahme 3 zu fixieren. Die Sondenaufnahme 3 kann zum Beispiel eine an ihrer Außenseite aufgeschweißte Schraubenmutter aufweisen, an der der Deckel 28 mit Hilfe einer Schraube befestigbar ist.

Um den Sondenhalter 1 im Fließgewässer noch besser fixieren oder sichern zu können, ist das Verbindungselement 22 mit einer Öffnung 33 versehen dargestellt. Die Öffnung 33 ist dabei beispielsweise in der Nähe eines Anströmungsrands, der beim Einsatz des Sondenhalters 1 entgegen einer Fließrichtung des Fließgewässers weist, angeordnet. An der Öffnung 33 ist beispielsweise ein Ankerelement anbringbar. Alternativ kann an der Öffnung 33 beispielsweise ein Seil angebracht werden, um den Sondenhalter 1 aus dem Fließgewässer herausziehen zu können oder es außerhalb des Fließgewässers zu befestigen und damit gegen Diebstahl zu schützen.

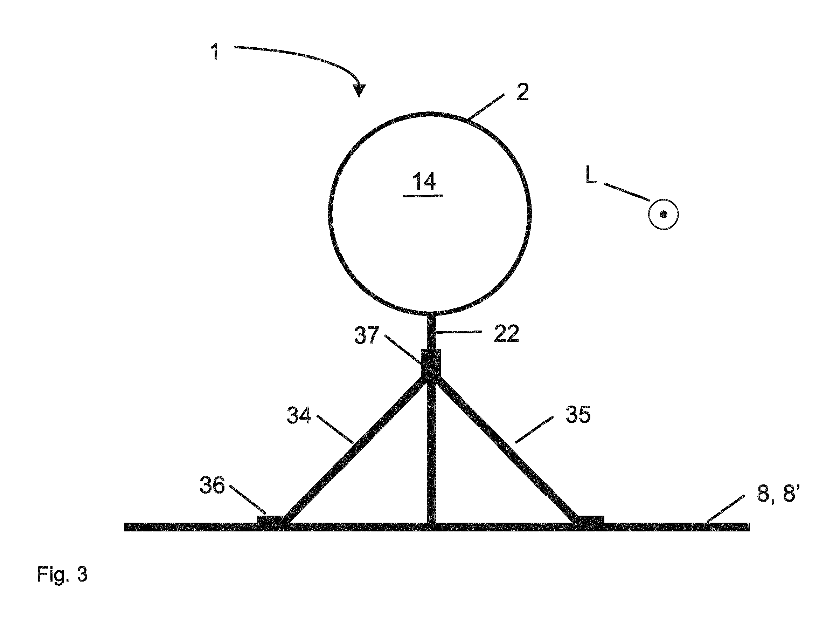

Figur 3 zeigt ein weiteres Ausführungsbeispiel des erfindungsgemäßen Sondenhalters 1 schematisch in einer Frontalansicht entgegen der Längsrichtung L. Für Elenente, die in Funktion und/oder Aufbau den Elementen der Ausführungsbeispiele der Figuren 1 oder 2 entsprechen, sind dieselben Bezugszeichen verwendet Der Kürze halber ist im Folgenden lediglich auf die Unterschiede zu den Ausführungsbeispielen der Figuren 1 und 2 eingegangen.

Figur 3 zeigt den Sondenhalter 1 mit der das verschlossene Ende 14 aufweisenden Sondenaufnahme 2, dem Abstellelement 8 und dem Verbindungselement 22. Um zu verhindern, dass sich die Sondenaufnahme 2 und womöglich auch die Sondenaufnahme 3 ohne weiteres quer zur Längsrichtung L relativ zum Abstellelement 8 bewegen lassen, ist der Sondenhalter 1 des Ausführungsbeispiels der Figur 3 mit einem Stützelement 34 und insbesondere mit zwei Stützelementen 34, 35 versehen. Quer zur Längsrichtung L ist das Verbindungselement 22 zwischen den Stützelementen 34, 35 angeordnet.

Im Folgenden ist lediglich das Stützelement 34 beschrieben. Selbstverständlich kann das Stützelement 35 wie das Stützelement 34 ausgebildet sein, wobei die Stützelemente 34, 35 vorzugsweise symmetrisch um das Verbindungselement 22 herum angeordnet sind.

Das Stützelement 34 ist beabstandet von dem Verbindungselement 22 am Abstellelement 8 befestigt. Am Verbindungselement 22 ist das Stützelement 34 beabstandet vom Abstellelement 8 und insbesondere zwischen dem Abstellelement 8 und der Sondenaufnahme 2 befestigt. Das Abstellelement 8, das Verbindungselement 22 und das Stützelement 34 bilden also ein dreieckiges Fach einer Fachwerkstruktur. Quer zur Längsrichtung L auf die Sondenaufnahme 2 und womöglich auch auf die Sondenaufnahme 3 wirkende Kräfte werden durch das Stützelement 34 effektiv in das Abstellelement 8 eingeleitet, ohne dass sich eine der Sondenaufnahmen 2, 3 nennenswert in Richtung auf das Abstellelement 8 zu neigen kann.

Quer zur Längsrichtung L kann das Stützelement 34 plattenförmig ausgebildet sein und eine Länge aufweisen, die der Länge des Aufnahmekanals des Abstellelements oder des Verbindungselements entspricht. Das plattenförmige Stützelement 34 ist beispielsweise trapezförmig ausgeformt, wobei die kürzere Grundseite vom Abstellelement 8 und längere Grundseite vom Aufnahmekanal 4 weg weisen.

Um eine punktuelle Verbindung des Stützelementes 34 mit dem Abstellelement 8 und/oder dem Verbindungselement 22 zu vermeiden, ist das Stützelement 34 vorzugsweise mit Fixierenden 36, 37 versehen, die jeweils flächig an dem Abstellelement 8 oder dem Verbindungselement 22 anliegend an diesem befestigt und beispielsweise mit diesem verschweißt sind. Die Fixierenden 36, 37 ermöglichen eine großflächige Verbindung zwischen dem Stützelement 34 und dem Abstellelement 8 sowie dem Verbindungselement 22, sodass quer zur Längsrichtung L auf die Sondenaufnahme 2, 3 einwirkenden Kräfte unter Vermeidung punktueller Belastungen großflächig abgeleitet werden können.

## Patentansprüche

1. Sondenhalter (1) zur Fixierung einer zylindrischen Sonde (5) in einem Fließgewässer, **gekennzeichnet durch** einen Aufnahmekanal (4) für die Sonde (5), wobei sich der Aufnahmekanal (4) **durch** ein in und entgegen einer Längsrichtung (L) des Aufnahmekanals (4) begrenztes Strömungsfenster (7) des Sondenhalters (1) erstreckt, das quer zur Längsrichtung (L) offen ist.

2. Sondenhalter (1) nach Anspruch 1, **dadurch gekennzeichnet, dass** sich der Aufnahmekanal (4) durch zwei voneinander beabstandet angeordnete hohlzylindrische Sondenaufnahmen (2, 3) erstreckt, die das Strömungsfenster (7) in und entgegen der Längsrichtung (L) begrenzen.

3. Sondenhalter (1) nach Anspruch 2, **dadurch gekennzeichnet, dass** ein von der anderen Sondenaufnahme (3) weg weisendes Ende (14) einer der Sondenaufnahmen (2) geschlossen ist.

4. Sondenhalter (1) nach Anspruch 2 oder 3, **dadurch gekennzeichnet, dass** ein von der anderen Sondenaufnahme (2, 3) weg weisendes Ende (14, 16) einer der Sondenaufnahmen (2, 3) gegen ein ungewolltes Öffnen gesichert verschließbar ist.

5. Sondenhalter (1) nach einem der Ansprüche 2 bis 4, **gekennzeichnet durch** ein Abstellelement (8), das mit den Sondenaufnahmen (2, 3) starr verbunden ist und auf dem der Sondenhalter (1) verkippsicher abstellbar ist.

6. Sondenhalter (1) nach Anspruch 5, **dadurch gekennzeichnet, dass** das Abstellelement (8) eine Ablagefläche (8') für Schüttgut aufweist, die den Sondenaufnahmen (2, 3) zu gewandt ist.

7. Sondenhalter (1) nach einem der Ansprüche 2 bis 6, **gekennzeichnet durch** ein Verbindungselement (22), an dem zumindest eine der Sondenaufnahmen (2, 3) und das Abstellelement (8) befestigt sind, wobei zumindest eine Sondenaufnahme (2, 3) beabstandet vom Abstellelement (8) am Verbindungselement (22) befestigt ist.

8. Sondenhalter (1) nach Anspruch 7, **dadurch gekennzeichnet, dass** das Verbindungselement (22) einen an einer den Sondenaufnahmen (2, 3) anliegenden Halteabschnitt (25, 26) und einen zwischen den Sondenaufnahmen (2, 3) angeordneten Strömungsabschnitt (27) aufweist, wobei sich der Strömungsabschnitt (27) entlang des Strömungsfensters (7) erstreckt und wobei der Strömungsabschnitt (27) einen größeren Abstand zum Aufnahmekanal (4) aufweist, als der Halteabschnitt (25, 26).

9. Sondenhalter (1) nach einem der Ansprüche 1 bis 8, **gekennzeichnet durch** wenigstens ein Stützelement (34, 35), das den Aufnahmekanal (4) quer zu seiner Längsrichtung (L) gegen Bewegungen sichert.

10. Sondenhalter (1) nach einem der Ansprüche 2 bis 9, **gekennzeichnet durch** einen Abstandshalter (11, 12, 13) zur Positionierung der Sonde (5) beabstandet zu einer Innenseite einer der Sondenaufnahmen (2, 3).
